# EUROPEAN PATENT SPECIFICATION

(11) **EP 3 636 281 B1**
(45) Date of publication and mention of the grant of the patent: **01.11.2023**
(21) Application number: 18798176.6
(22) Date of filing: 08.05.2018
(51) Int. Cl.: A61K 45/00, A61K 31/00, A61P 25/24, G01N 33/487, G01N 33/50, G01N 33/68, A61K 31/135, A61K 31/4015, A61K 31/4184, A61K 31/495, A61K 45/06

(54) **METHOD FOR TREATING DEPRESSION, AND PHARMACEUTICAL COMPOSITION**
VERFAHREN ZUR BEHANDLUNG VON DEPRESSION UND PHARMAZEUTISCHE ZUSAMMENSETZUNG
MÉTHODE DE TRAITEMENT DE LA DÉPRESSION, ET COMPOSITION PHARMACEUTIQUE

(30) Priority: 09.05.2017 CN 201710322647; 09.05.2017 CN 201710322266; 09.05.2017 CN 201710322646
(43) Date of publication of application: 15.04.2020
(73) Proprietor: Zhejiang University, Hangzhou, Zhejiang 310058 (CN)
(72) Inventor: HU, Hailan, Hangzhou Zhejiang 310058 (CN); YANG, Yan, Hangzhou Zhejiang 310058 (CN); CUI, Yihui, Hangzhou Zhejiang 310058 (CN)
(74) Representative: Brevalex
(86) International application number: PCT/CN2018/086043
(87) International publication number: WO 2018/205935

(56) References cited:
- WO-A2-2007/111880
- CN-A- 102 512 682
- CN-A- 106 562 952
- US-A- 4 694 010
- US-A1- 2016 375 000
- KRAUS ET AL: "Trazodone inhibits T-type calcium channels", NEUROPHARMACOLOGY, PERGAMON PRESS, OXFORD, GB, vol. 53, no. 2, 6 August 2007 (2007-08-06) , pages 308-317, XP022188033, ISSN: 0028-3908, DOI: 10.1016/J.NEUROPHARM.2007.05.011
- HENRIKE HARTUNG ET AL: "High-frequency stimulation of the subthalamic nucleus modulates neuronal activity in the lateral habenula nucleus", EUROPEAN JOURNAL OF NEUROSCIENCE., vol. 44, no. 9, 13 October 2016 (2016-10-13), pages 2698-2707, XP055733278, GB ISSN: 0953-816X, DOI: 10.1111/ejn.13397
- ZEYNEP ATES-ALAGOZ et al.: "NMDA Receptor Antagonists for Treatment of Depression", Pharmaceuticals, vol. 6, no. 4, 3 April 2013 (2013-04-03), pages 480-499, XP055479845, ISSN: 1424-8247
- CAO, Qiuyun: "The Clinical Features and Treatment of Late-Onset Depression", Chinese Journal of Brain Diseases and Rehabilitation, vol. 5, no. 3, 30 June 2015 (2015-06-30), pages 141-144, XP009517789, ISSN: 2095-123X
- Yang Hongbo; Zhang Jichuan: "Research Progress on Effect of Lateral Habenular Nucleus in Depression", Chinese Journal of Gerontology, vol. 36, no. 5, 31 March 2016 (2016-03-31) , pages 1246-1248, XP009517781, ISSN: 1005-9202

## Description

### FIELD OF THE INVENTIONS

The invention relates to the field of disease therapy and pharmacy field. In particular, the invention provides a pharmaceutical compositions for use in a method of treating depression.

### BACKGROUND

Depression has a high incidence in the population. If left untreated, depression may be debilitating emotionally and physically. Depressive disorders include a range of symptoms, as defined and described by the US National Institute of Mental Health as follows: "Persistent sad, anxious or" empty "mood; a sense of hopelessness, a sense of pessimism guilt; worthlessness, helplessness; lose once enjoyed hobbies and activities (including sexual) interest or pleasure; energy reduction, fatigue; difficulty concentrating, memory difficulties, it is difficult to make a decision; hyperactivity, irritability.

Lateral habenula (LHb), the lateral part of habenula, together with the pineal gland forms the epithalmus. LHb is a gateway from the limbic forebrain to midbrain aminergic nucleus. LHb negative regulate both the dopamine and serotonin systems through either direct or indirect innervations, which play essential roles in a wide range of drug addiction, reward and avoidance, pain, sleep and a variety of psychiatric disorders.

Evidence from human studies and animal models has surged to associate the LHb with major depressive disorder. In both acute learned helplessness (aLH) and congenital learned helplessness (cLH) rats, VTA-projecting habenula neurons demonstrate enhanced synaptic activity (Li, B. et al. Nature 470, 535-539, 2011). In normal state, LHb has relative weak inhibition on VTA and DRN. In depressed state, stress causes upregulation of CaMKII level, which leads to increased membrane trafficking of GluR1, increased synaptic efficacy and spike output of LHb neurons. As a result, the LHb inhibition onto VTA and DRN are enhanced, causing anhedonia and behavioral despair (Li et al., Science 341, 1016-1020, 2013). Kraus et al. disclose an anti-depressive effect of calcium channel inhibitors, naming tradozone and Mibefradil and patch-clamp analysis of neuronal cells in brain slices showing reduced bursts in the cells after treatment with the blocker.

There are already some commonly used antidepressants in the field, but these drugs usually take effect after a long period of time. Moreover, the pathological mechanism leading to depression has not been fully recognized. There is a need in the art for new methods and drugs for treating depression, which have a faster onset rate or a safer effective dosage.

### SUMMARY

The present inventions provides a pharmaceutical composition for use in a method of treating depression in a subject, wherein the depression is characterized by having an increased burst firing in neurons of a lateral habenula of the subject, wherein the pharmaceutical composition is administered locally into the lateral habenula of the subject, the pharmaceutical composition comprising a reagent that is capable of inhibiting the burst firing, but is incapable of inhibiting tonic pulse, in the neurons of the lateral habenula, wherein the reagent comprises one selected from: ketamine; AP5; Mibefradil; a combination of ketamine and Mibefradil; or a combination of AP5 and Mibefradil. In a further aspect the present invention relates to a pharmaceutical composition for use in a method of treating depression in a subject, wherein the depression is characterized by having an increased burst firing in neurons of a lateral habenula of the subject, wherein the pharmaceutical composition is administered locally into the lateral habenula of the subject, the pharmaceutical composition comprising a reagent that is capable of inhibiting the burst firing, but is incapable of inhibiting tonic pulse, in the neurons of the lateral habenula, wherein the reagent comprises a combination of ketamine and ethosuximide, wherein ketamine has a dose of 2.5 mg/kg and ethosuximide has a dose of 100 mg/kg. Further embodiments are defined by the appended dependent claims.

Subjects in need of the methods and medicaments (pharmaceutical compositions) described herein include subjects diagnosed with depression. The subject to be treated can be a mammal, including a human or a non-human primate such as a monkey. The mammal can be other animals such as rats, mice, rabbits, pigs, dogs, and the like. The mammal can be a domestic animal such as a cat or a dog.

The term 'burst', or `burst firing', means a neuronal firing pattern with two or more spikes of plateau potentials (short as spikes hereafter) each time of firing.

The term "inhibiting burst firing", "inhibiting burst", "inhibition of burst" means inhibiting the level of neuronal burst firing, including decreasing of burst frequency or reducing a number of intra-bursting spikes, reducing bursting amplitude and even eliminating burst firing.

The term `tonic pulse' or 'tonic firing' means a neuronal firing pattern with only one spike in each firing.

An agent inhibiting burst in lateral habenula includes a compound, a complex or a mixture capable of inhibiting burst, as well as reagent used in a method for inhibiting burst (including a surgical method), and the like. The reagent can be a small molecule compound or complex, or a macromolecular active component such as a protein or a nucleic acid, for example, an antagonist such as an antibody that binds to a protein in the burst related physiological pathway, or a nucleic acid that affects the expression level of such proteins.

As used herein, "treatment" comprises any of an ongoing process or outcome that ameliorates, palliates, decreases or prevents the symptoms associated with a medical condition or infirmity; an ongoing process or outcome that improves the symptoms associated with a medical condition or infirmity; an ongoing process or outcome to normalize body functions in disease or disorders that result in impairment of specific bodily functions; or an ongoing process or outcome that elicits an improvement in one or more of the clinically measured parameters of the disease. A treatment objective is to prevent or slow down (lessen) an undesired physiological condition, disorder or disease, or to obtain beneficial or desired result. The result can be, e.g., medical, physiological, clinical, physical therapy, occupational therapy, subjective to a health care worker or to a patient; or a parameter understood in the art as a "quality of life" or an "activity of daily living". Beneficial or desired clinical results comprise, but are not limited to, alleviation of symptoms; diminution/diminishment of the extent of the condition, disorder or disease; stabilization (i.e., not worsening) of the state of the condition, disorder or disease; delay in onset or slowing of the progression of the condition, disorder or disease; amelioration or palliation of the condition, disorder or disease; and remission (whether partial or total), whether detectable or undetectable; or enhancement or improvement of the condition, disorder or disease. In one aspect, treatment includes eliciting a clinically significant response without excessive levels of side effects. In one aspect, treatment also includes prolonging survival as compared to expected survival if not receiving treatment. In one aspect, treatment refers to the administration of medicine or the performance of medical procedures with respect to a patient. As used herein, treatment can be to prophylax (prevention), to cure the infirmity or malady, or to ameliorate the clinical condition of the patient, including a decreased duration of illness or severity of illness, or subjective improvement in the quality of life of the patient or a prolonged survival of the patient.

N-methyl-D-aspartate is an excitatory amino acids , EAA, which is an excitatory neurotransmitter of the central nervous system. The N-methyl-D-aspartate receptor (NMDA receptor or NMDAR) is an ionic receptor involved in excitatory synaptic transmission. Modulation of NMDA receptors modulates glutamatergic neurotransmitter mediated neurological effects.

N-methyl-D-aspartate receptor inhibitors disclosed herein include but not limited to:
1) competitive NMDA receptor inhibitors (inhibitors that compete with glutamate binding sites), such as AP5, AP7, CPPene, Selfotel;
2) non-competitive NMDA receptor inhibitors (inhibitors that block allosteric binding sites), such as Aptiganel, ketamine, memantine, Huperzine A, Ibogaine, HU-211, Gabapentin, PD-137889;
3) non-competitive NMDA receptor channel blockers (channel blocker), such as Amantadine, Atomoxetine, AZD6765, Dextromethorphan, memantine hydrochloride, MK801(Dizocilpine); or
4) glycine binding site inhibitors, such as TK-40, Kynurenic acid.

A T-type calcium channel is also known as transient calcium channel, or low voltage activate calcium channel. The T-type calcium channel plays an important role in modulating excitations in central and peripheral nervous system. In vertebrates, T-type calcium channel consists of three different α1 subunit genes: CACNA1G, CACNA1H and CACAN1I, encoding α1G, α1H and α1I respectively, which respectively form three subtypes of T-type calcium channels: Cav3.1, Cav3.2, and Cav3.3. A T-type calcium channel is typically a tetramer protein, with each monomer (i.e. α1 subunit) containing four homologous domains. The poreof the channel is composed of the four homologous domains. Pole helices and the terminal of extracellular S6 fraction connect each other to thereby form a selective filter for calcium ions. A positive-charged amino acid residue is present every third amino acid in each S4 fraction of the homologous domain, thereby constituting the channel voltage sensor which controls the switch-on or switch-off of the channel when the membrane potential changes.

Herein, the T-type calcium channel inhibitors disclosed herein include, but are not limited to: succinimides (such as ethosuximide and methsuximide), hydantoins, zonisamide, valproate sodium, phenytoin, Mibefradil, Phenytoin, sipatrigine, piperazine analogues (such as Flunarizine and Z941), piperidine analogues (such as Z944 or Fluoropiperidine), TTA-P1, TTA-P2, quinazolinone, Pimozide, Trimethadione or Dimethadione, TTA-Q4, and ML218, etc.

In the present disclosure, the term depression may especially refer to lateral-habenula-mediated depression, particularly, refers to lateral-habenula-burst-mediated depression. The authors of the disclosure have found and demonstrated that the abnormal firing of neurons in the lateral habenula, especially the abnormal burst firing plays an important role in the generation of depression. The authors of the disclosure have also identified key factors affecting the burst in the lateral habenula, and that, by regulating these key factors, depression can be inhibited or eliminated.

Anti-depression drugs described herein include, but are not limited to, the following types of drugs:
- melatonin agonists
- selective serotonin reuptake inhibitors (SSRIs)
- serotonin and norepinephrine reuptake inhibitors (SNRIs)
- monoamine oxidase inhibitors (MAOIs)
- tricyclic antidepressants (TCAs)
- triple monoamine uptake blockers
- metabotropic glutamate receptors (of mGluRs)
- GABA antagonist
- NK1 antagonist
- NK2 antagonist
- CRF1 antagonist
- arginine vasopressin V1b antagonists
- MCH receptor antagonists
- NT-3 antagonist, NT-4 antagonists
- CREB antagonist

The above types of antidepressants and specific drugs are listed in WO2007/137247.

Certain NMDA receptor inhibitors are known in the art for use in the treatment of depression. However, in these reports, the anti-depression mechanism discovered or presumed are completely different from the mechanism discovered by the present invention, that is, by inhibiting the abnormal firings of lateral habenula neurons, particularly the abnormal burst. Without affecting the novelty and inventive steps of the present invention, in one aspect of the present invention, in the method for treating depression by inhibiting burst in the lateral habenula, the use of the reagent for inhibiting burst in the lateral habenula for the manufacturing a medicament for treating depression, and the pharmaceutical composition for treating depression, said reagent for inhibiting burst does not include these NMDA receptor inhibitors, such as AP5, CPPene, MK801, memantine, ketamine, felbamate, glycine, D-serine, D-cycloserine, L-glutamic acid efendil, and the like.

Certain compounds are known in the art to be able to inhibit T-type calcium channel, and they show certain anti-depression phenomenon, such as Fluoxetine, trazodone, ethosuxamine, trimethyldione, sodium valproate, pimozide and zonisamide. However, in these reports, the anti-depression mechanism discovered or presumed are completely different from the mechanism discovered by the present invention, that is, by inhibiting the abnormal firings of lateral habenula neurons, particularly the abnormal burst. Without affecting the novelty and inventive steps of the present invention, in one aspect of the present invention, in the method for treating depression by inhibiting burst in the lateral habenula, the use of the reagent for inhibiting burst in the lateral habenula for the manufacturing a medicament for treating depression, and the pharmaceutical composition for treating depression, said reagent for inhibiting burst does not include Fluoxetine, trazodone, ethosuxamine, trimethyldione, sodium valproate, pimozide or zonisamide. methods and medicaments used on nervous tissues, particularly on those in the brain, such as the lateral habenula, it is beneficial to limit the effects of the drug or medicine to the target tissue. The administration of a medicine locally in the lateral habenula is a limiting technical feature of a method or a reagent for treating depression. In any method or medicament for LHb, whether the method or drug can take effects in LHb shall be considered, including whether the drug can reach LHb, and whether the effective concentration can be achieved in LHb, etc. According to the present disclosure, the medicament or pharmaceutical composition can have a dosage form allowing for local administration to the lateral habenula. The action of the medicament can be limited to the target tissue by local administration, for example by formulating the medicament as a dosage form that can be administered locally to the lateral habenula by cannulation or by sleeve implantation. In another example, the drug can be formulated as a dosage form having sustained release after being implanted into the tissue. The above medicaments can also be formulated in the form of tissue-specific targeted drug delivery systems. For example, a small molecule compound or a biologically active molecule (e.g. nucleic acid such as a protein-encoding DNA or mRNA molecule, or a protein such as an antibody, etc.) capable of specifically binding to a protein expressed in the lateral habenula can be conjugated with an antibody or an antibody fragment that binds to cells of the lateral habenula to thereby form a complex molecule capable of recognizing and binding to lateral habenula. antidepressants in the field generally take a week to several weeks to exert antidepressant effects. For example, the commonly used 5-HT reuptake inhibitors (SSRI) are usually effective in 2-3 weeks; 5-HT and serotonin and norepinephrine reuptake inhibitors is usually only effective after 1 week. The anti-depression methods and the medicaments or pharmaceutical compositions provided by the present invention have an onset time of less than one week, preferably less than three days, more preferably less than one day, such as less than 12 hours. The medicament provided by the present invention is also suitable as a fast-onset and an intermediate-acting or long-acting medicament for treating (inhibiting) depression, wherein a single-dose anti-depression effect can last for more than one day, preferably for more than three days, more preferably for more than one week.

While the active ingredient in the pharmaceutical compositions of the invention for use in therapy may be administered in the form of the raw compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

A therapeutically effective dose refers to that amount of active ingredient, which ameliorates the symptoms or condition. Therapeutic efficacy and toxicity, e.g. ED50 and LD50, may be determined by standard pharmacological procedures in cell cultures or experimental animals. The dose ratio between therapeutic and toxic effects is the therapeutic index and may be expressed by the ratio LD50/ED50.

The dose administered must of course be carefully adjusted to the age, weight and condition of the individual being treated, as well as the route of administration, dosage form and regimen, and the result desired, and the exact dosage should of course be determined by the practitioner.

The actual dosage depends on the nature and severity of the disease being treated, the exact mode of administration and form of administration, and is within the discretion of the physician, and may be varied by titration of the dosage to the particular circumstances of this invention to produce the desired therapeutic effect. However, it is presently contemplated that pharmaceutical compositions containing of from about 0.1 to about 1000 mg of active ingredient per individual dose, preferably of from about 1 to about 750 mg are suitable for therapeutic treatments.

### BRIEF DESCRIPTION OF THE DRAWINGS

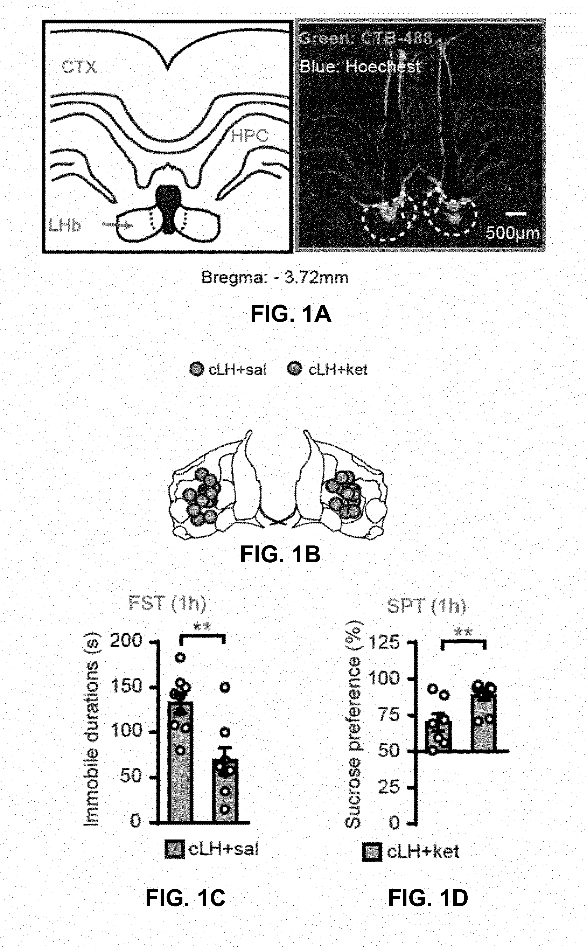

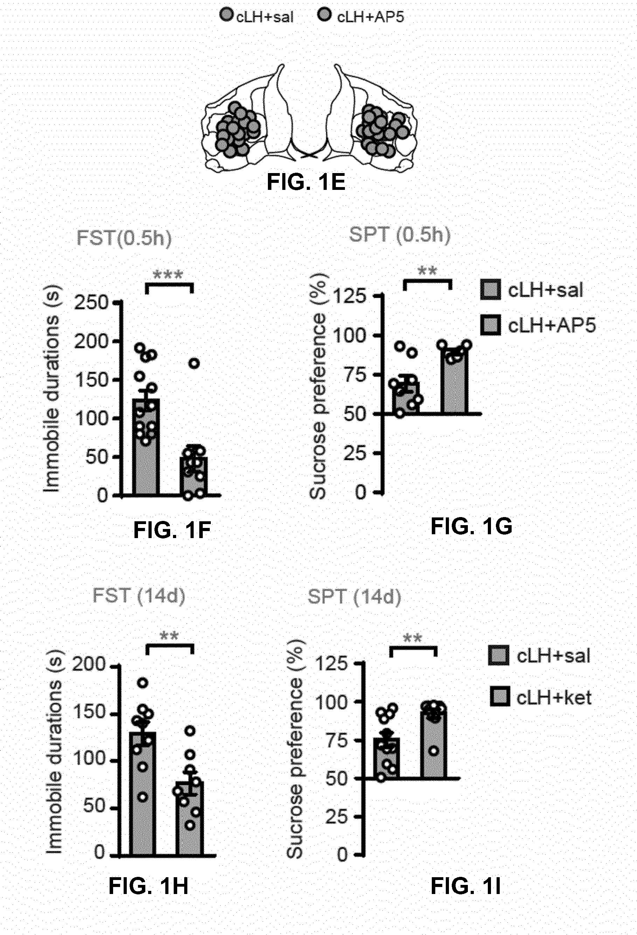

FIG. 1 shows local blockade of NMDARs in LHb is sufficient to elicit rapid and sustained antidepressant effects. (A) Illustration of bilateral implantation of cannulae in LHb of cLH rats. white dashed lines indicate location of habenula. (B-G) Acute antidepressant effects of local bilateral infusion of ketamine (25ug each side, 1h, B-D), AP5 (40 nmol each side, 0.5h, E-G) in LHb in forced swim test (FST, C, F) and sucrose preference test (SPT, D and G). (H-I) Sustained antidepressant effects of local bilateral infusion of ketamine lasted till 14 days. Data are mean ± s.e.m. *P < 0.05, **P < 0.01, ***P < 0.001, ****P < 0.0001, n.s. not significant.

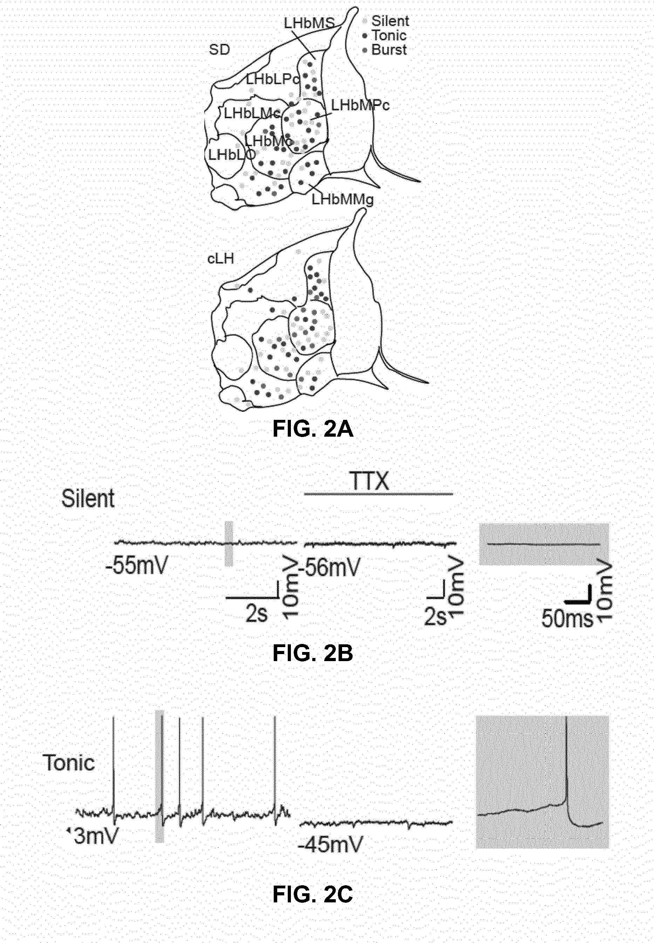

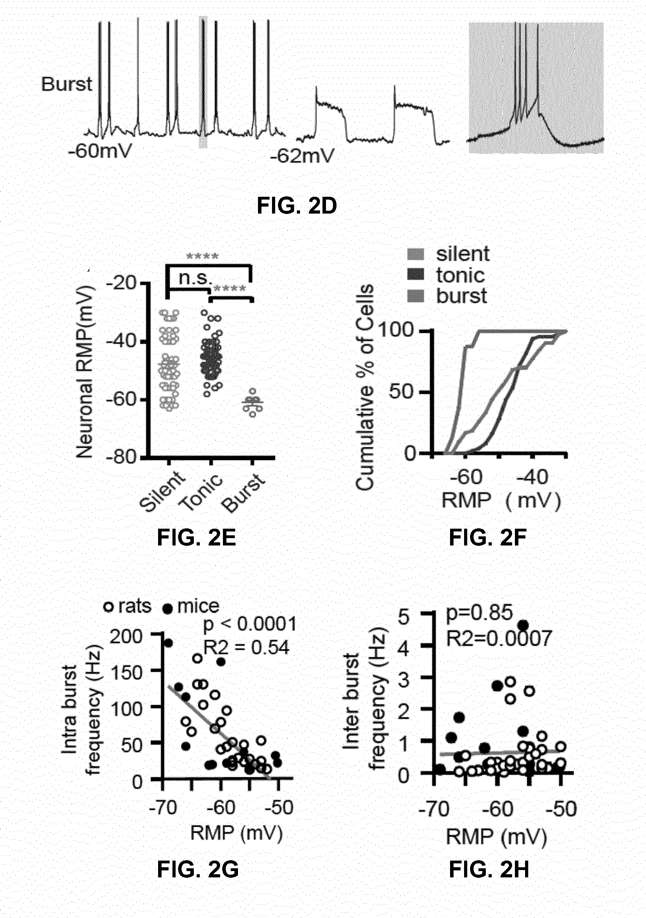

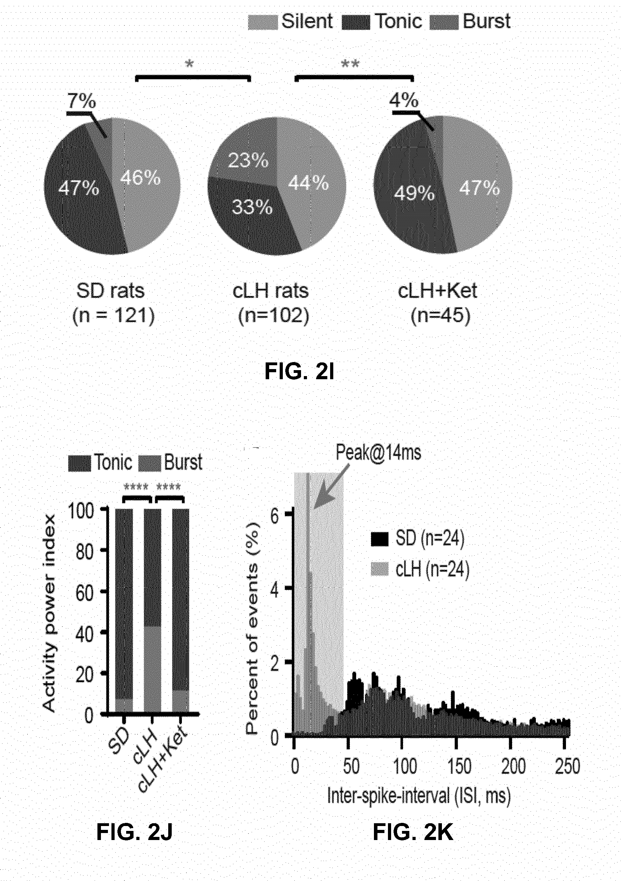

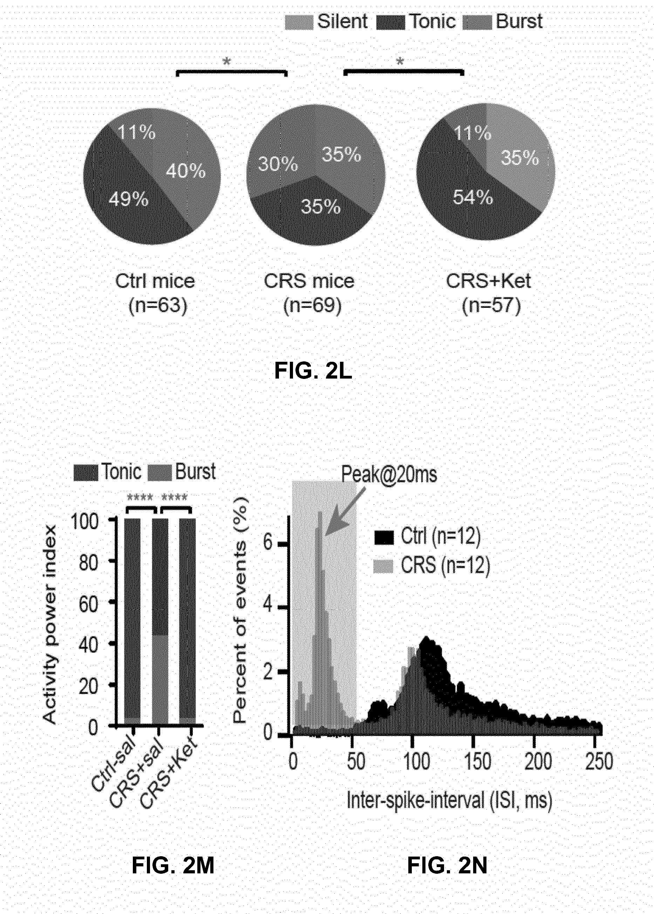

FIG. 2 shows bursting activity is enhanced in rat and mouse model of depression, which is reversed by ketamine. (A) Whole cell patch recordings sites across different subregions of LHb. (B-D) Representative traces showing spontaneous activity of three LHb neuron types, namely silent (B), tonic (C), and burst (D) firing types. Areas shown at the right with enlarged time scales to illustrate shape of spike pattern. Shown in the middle are responses of same neurons after TTX treatment, TTX is able to block spike of tonic or burst firing. (E-F) Scattered plots (E) and cumulative curves (F) denoting the mean and distribution of resting membrane potentials (RMPs). (G-H) Intra burst frequency but not inter burst frequency reversely correlated with RMPs. (I-N) Bursting neurons are significantly increased in cLH rat model (I-K) and chronic restraint stress (CRS) mouse model (I-N) of depression. (I, L) Pie charts illustrating the percentage abundance of the three types of LHb neurons in rats and micedepression model. (J, M) Bar graph illustrating the percentage of burst- and tonic-type spikes in all spikes recorded. (K, N) Histogram of inter-spike intervals (ISI, ms) distribution.

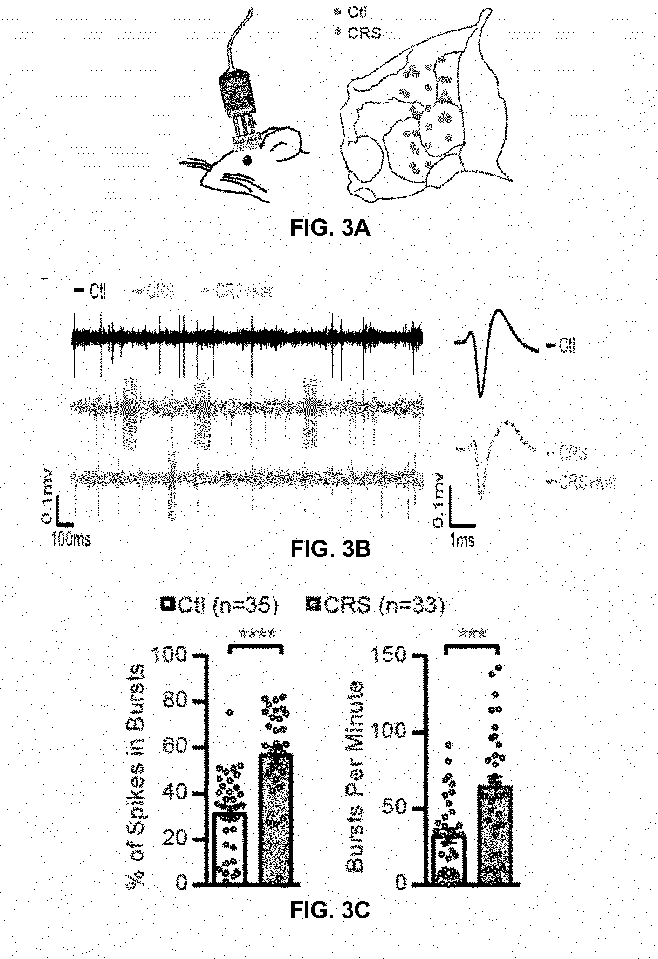

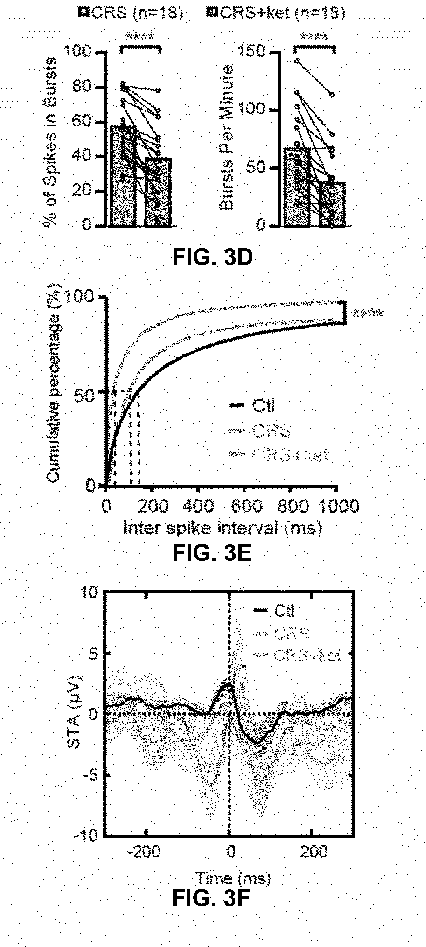

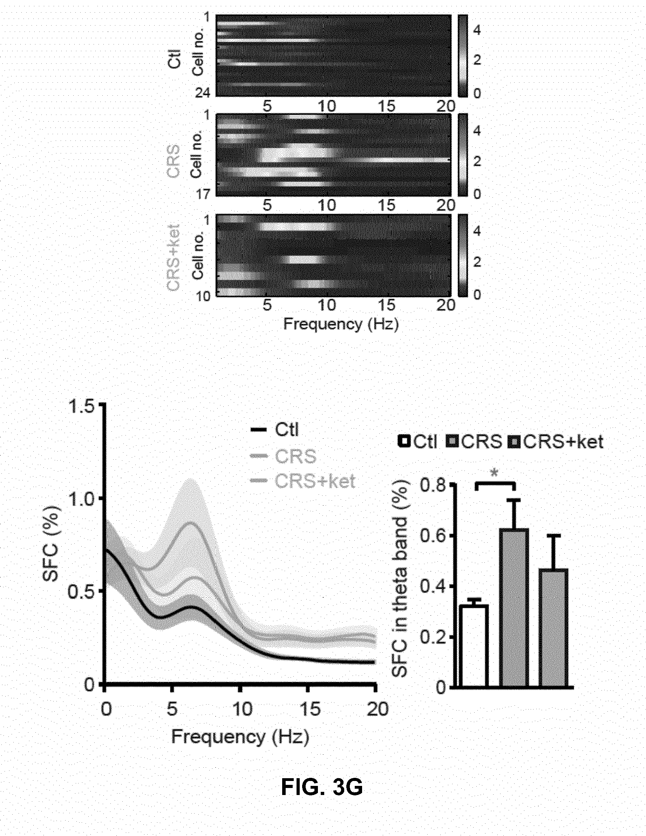

FIG. 3 shows ketamine suppresses enhanced LHb bursting activity and theta-band synchronization in vivo in chronic restraint mice. (A) Recording sites of each tetrode track in LHb of CRS and control mice. (B) Example traces (left) and averaged spike waveform (right) of recorded neurons from LHb of control (top), CRS (middle) and the same CRS unit after ketamine injection (bottom). Bursts are identified by ISI. (C-D) Percent of spikes in bursting mode and number of bursts per minute of neurons of CRS mice are significantly higher than thost of control, which are reversed by administration of ketamine. (E) Cumulative distribution of ISI from control, CRS mice, and CRS mice treated with ketamine. Dashed lines indicate the 50% percentile of ISI (control: 143ms; CRS: 33ms, CRS+ketamine: 121 ms). (F) Spike-triggered averages (STAs) of neurons recorded from control, CRS, and CRS mice after ketamine injection. Note the distance between the neighboring troughs is around 140 ms (corresponding to 7Hz) in CRS mice. (G) Spike-field coherence (SFC) of neurons recorded from control, CRS, and CRS mice after ketamine injection. Left: SFC of each unit; Middle: average SFC; Right: percent SFC in theta band (4-10Hz). Data are mean ± s.e.m. *P < 0.05, **P < 0.01, ***P < 0.001, ****P < 0.0001. n.s. not significant.

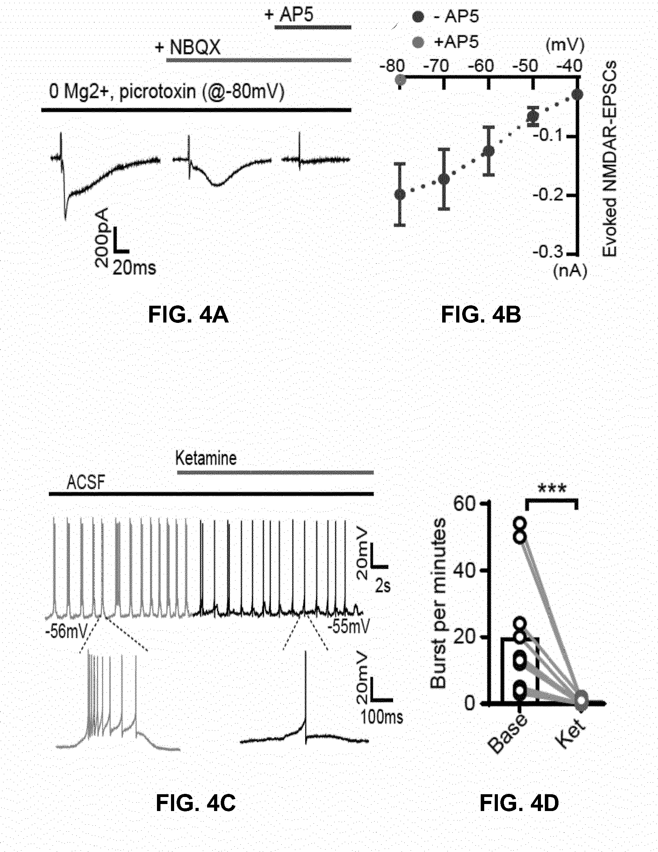

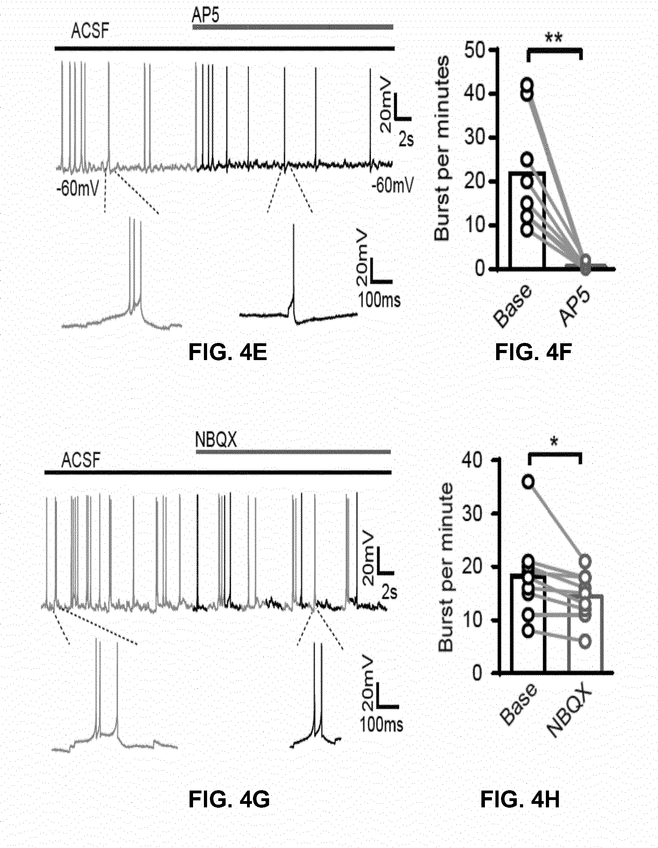

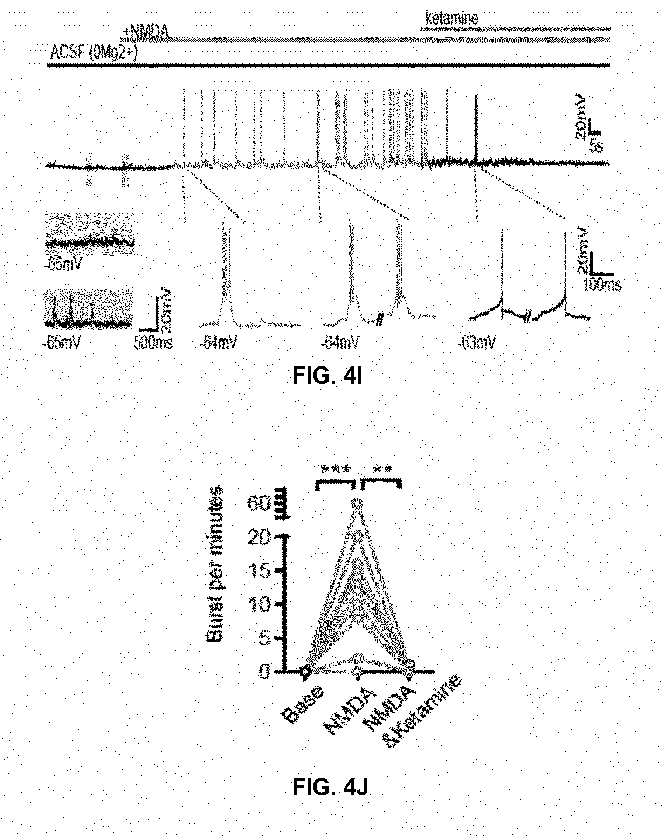

FIG. 4 shows LHb bursting requires activation of NMDARs. (A) Example traces showing evoked EPSCs when the cells are held at -80m V. NMDAR-EPSCs are isolated by application of NMDAR inhibitor picrotoxin and AMPA inhibitor NBQX in Mg²⁺ free ACSF, and confirmed by NMDAR inhibitor AP5 blockade. (B) Amplitudes of NMDAR-EPSCs under different voltages; the isolated NMDAR-EPSCs is completely blocked by AP5. (C-H) Example traces (left) and statistics (right, sampled within 1 min after drug application) showing effects of ketamine (C-D), AP5 (E-F) or NBQX (G-H) on spontaneous bursts in LHb. The left is a representative diagram. The right is a statistic diagram. (I-J) An originally silent LHb neurons induced to bursts by NMDA perfusion and returned to silence after ketamine application. Note that NMDA induces both large EPSP and bursting discharges. Data are mean ± s.e.m., *P < 0.05, **P < 0.01, ***P < 0.001, ****P < 0.0001. n.s. not significant.

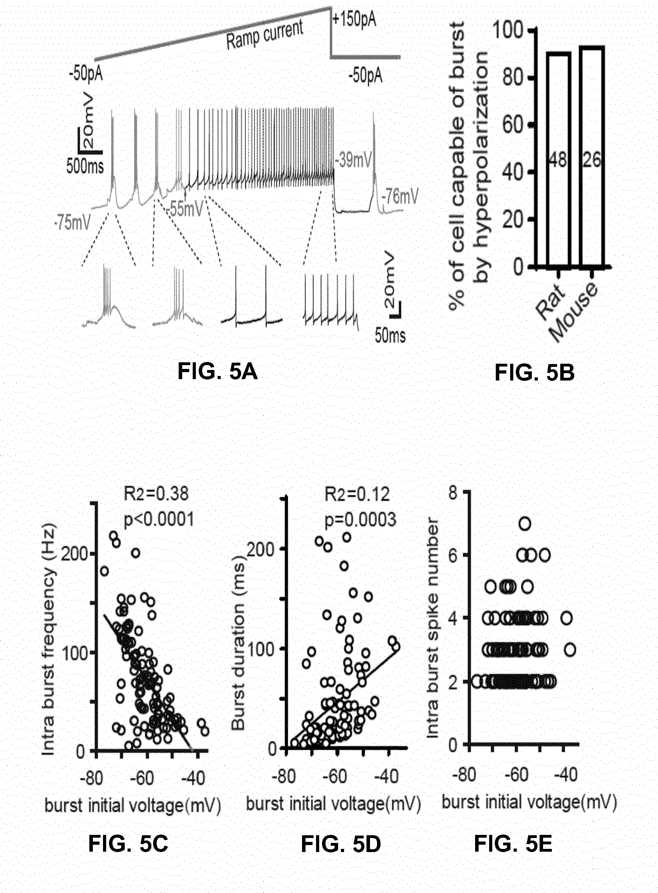

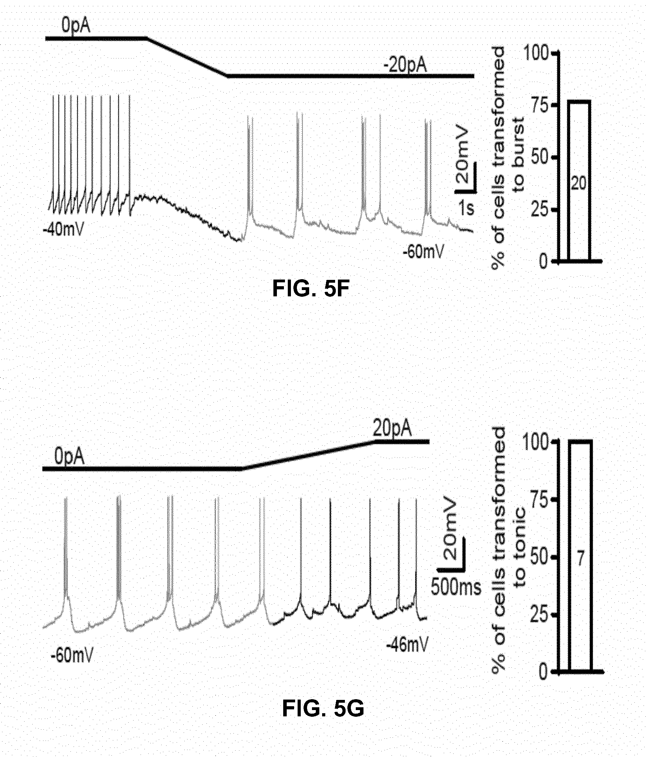

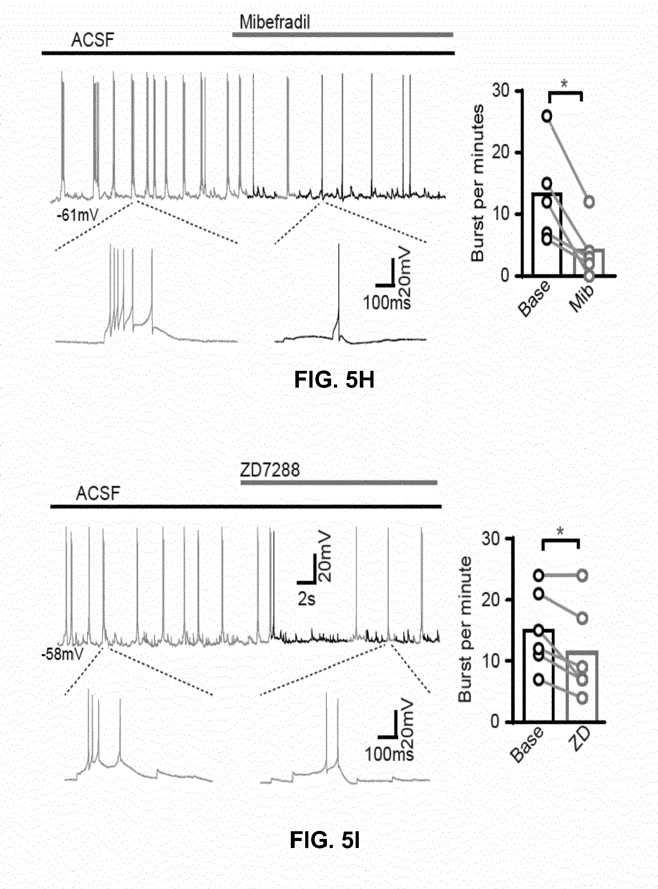

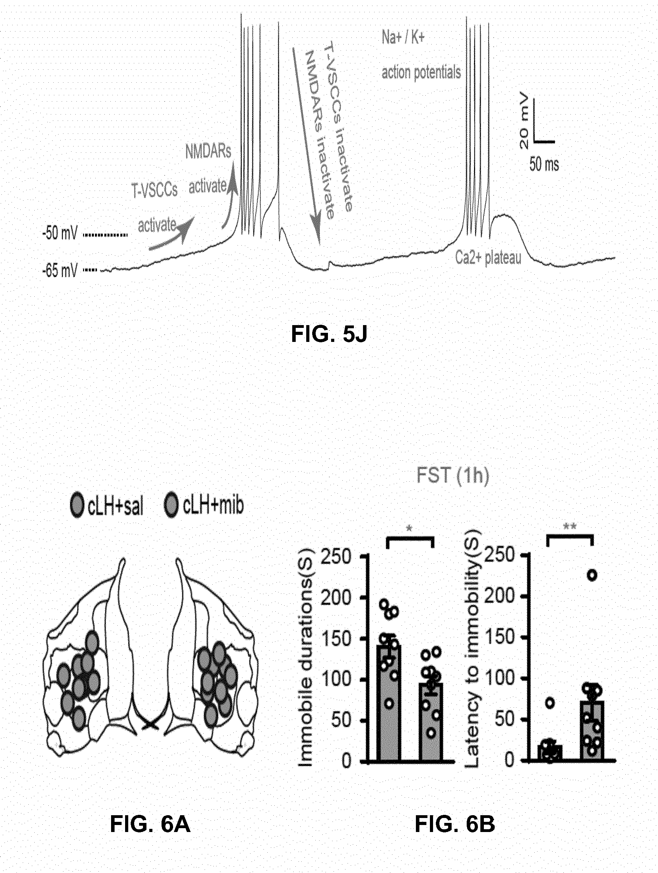

FIG. 5 shows LHb bursting requires membrane hyperpolarization and T-VSCCs. (A) Representative trace of a LHb neuron transformed from bursting- to tonic- firing mode with a ramp-like current injection, showing bursting at more hyperpolarized potential and tonic firing at more depolarized membrane potential. (B) Percentage of LHb neurons that can be induced into bursting mode with a hyperpolarizing current injection during the current ramp. (C-E) Correlations of membrane potential versus inner burst frequency (C), burst duration (D) and inner burst spike number (E) generated by current ramps. (F, G) Example trace (left) and statistics (right) of a spontaneously tonic-firing neuron transformed to burst-firing mode by hyperpolarization (F), and a spontaneously bursting neuron transformed to tonic firing by depolarization (G). (H, I) Example traces (left) and statistics (right, sampled within 1 min after drug application) showing effects of T-VSCC blocker miberfradil (H) or HCN blocker ZD7288 (I) on spontaneous bursts in LHb. (1) An example trace summarizing the ionic components and channel mechanisms involved in LHb bursting. Activation of T-VSCCs removes the Mg ion blockade of NMDARs. The opening of these two channels synergistically drive membrane potential toward the threshold for a burst of APs. After the quick inactivation of T-VSCCs and NMDARs, the return of RMP back to below -55mV de-inactivates T-VSCCs, which initiates another cycle of burst. Data are mean ± s.e.m., *P < 0.05, **P < 0.01, ***P < 0.001, ****P < 0.0001. n.s. not significant.

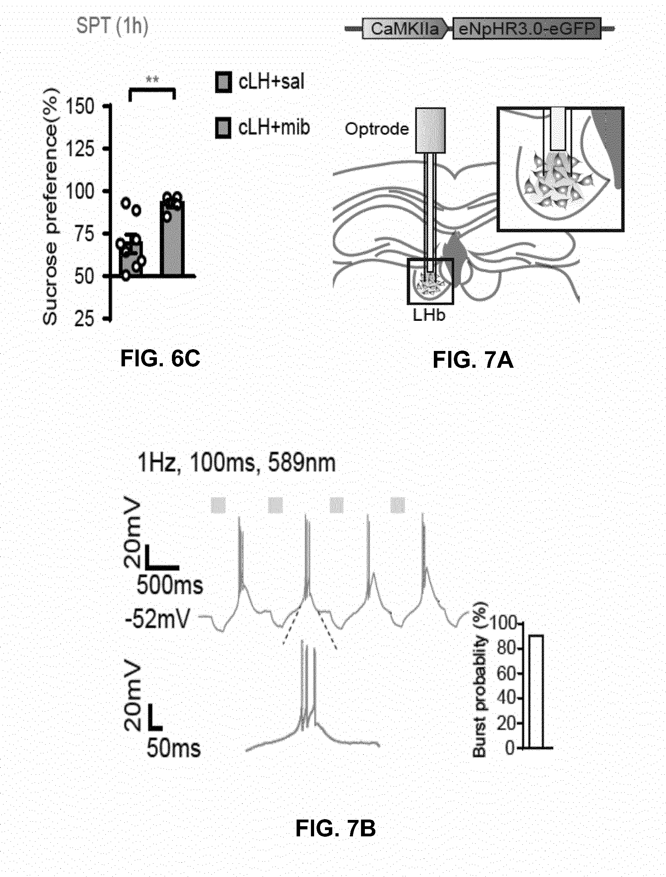

FIG. 6 shows local bilateral infusion of mibefradil in LHb exerts a rapid antidepressant effect in FST (B) and SPT (C). Infusion sites are verified by CTB (A). Data are mean ± s.e.m., *P < 0.05, **P < 0.01, n.s. not significant.

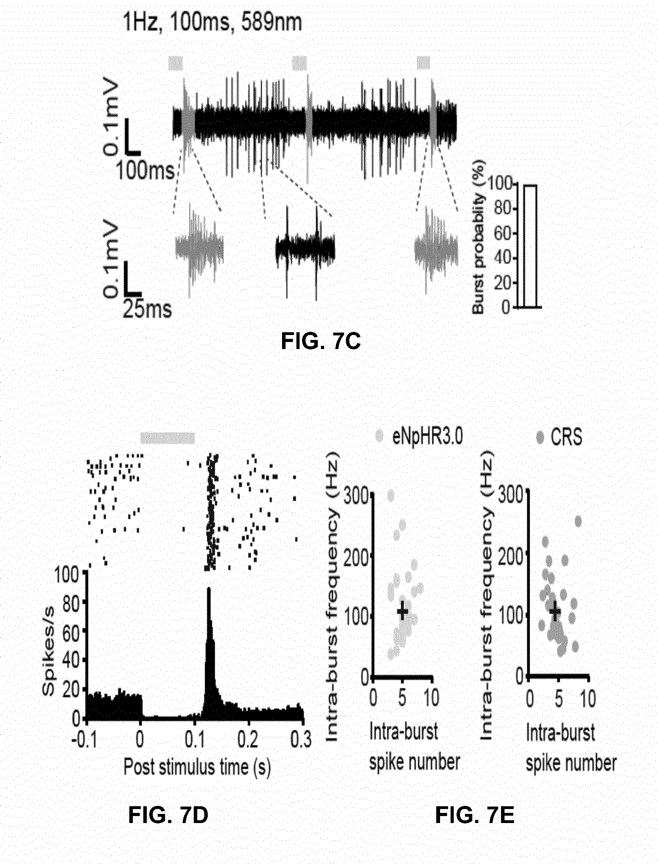

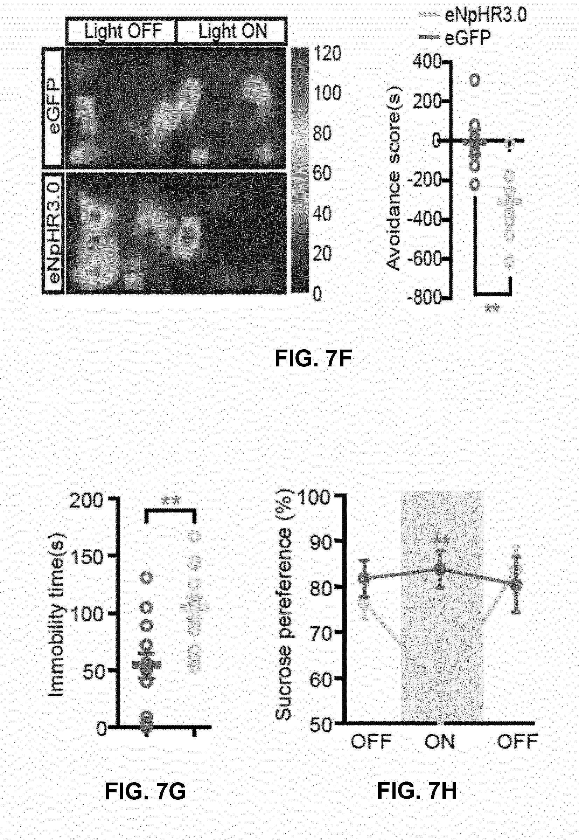

FIG. 7 shows eNpHR-induced rebound burst drives behavioral aversion and depressive-like symptoms. (A) Construct of AAV2/9-eNpHR3.0 (top), and illustration of optrode recording (bottom). (B, C) Representative traces showing rebound bursts reliably elicited by pulsed yellow light in LHb brain slices in vitro (B) and in vivo from mice infected with AAV2/9-eNpHR3.0 (C). Percentage of successfully induced burst is shown on right. (D) Raster plots (top) and post-stimulus time histogram (bottom) of an example LHb neuron responding to 100ms yellow light stimulation from in vivo optrode recording. (E) Distribution of intra burst frequencies and intra burst spike numbers of eNpHR3.0-driven rebound bursts (left) are comparable to those in CRS mice (right). Means are represented by the black crosses. (F) Real-time place aversion (RTPA) induced by eNpHR3.0-driven bursts (F). Left: representative heat maps of RTPA; Right: quantitative aversion score (see Methods). (G, H) Depressive-like behaviors in FST(G) and SPT (H) induced by eNpHR3.0-driven bursts. Data are represented as mean ± s.e.m., *P < 0.05, **P < 0.01, ***P < 0.001, ****P < 0.0001. n.s. not significant.

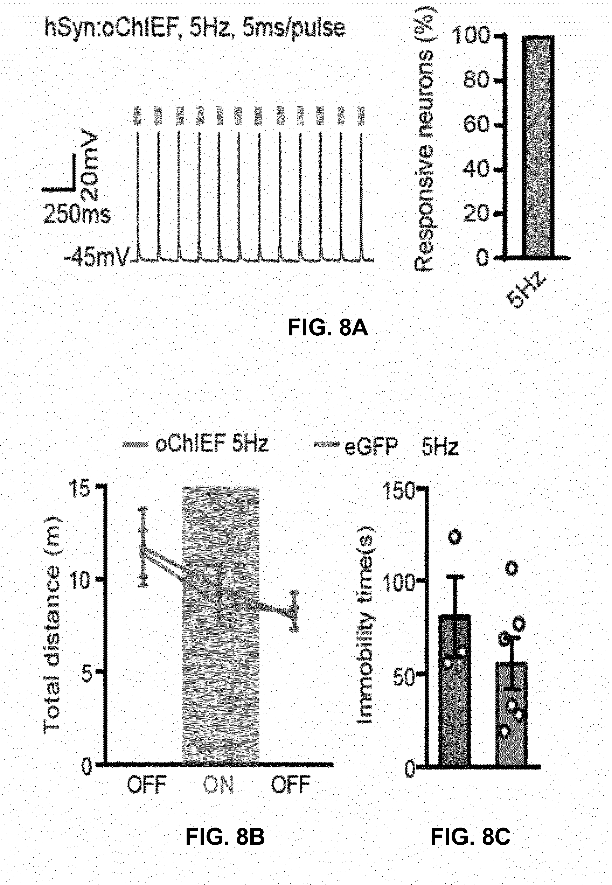

FIG. 8 shows stimulation yielded the same overall firing rate as the rebound burst protocol do not cause depressive-like phenotypes. (A) Representative trace showing LHb neurons following a 5Hz tonic blue light stimulation protocol in LHb brain slices. (B, C) 5Hz photostimulation of mice expressing oChIEF does not change locomotion in OPT (B), and does not induce depressive phenotypes in FST (C).

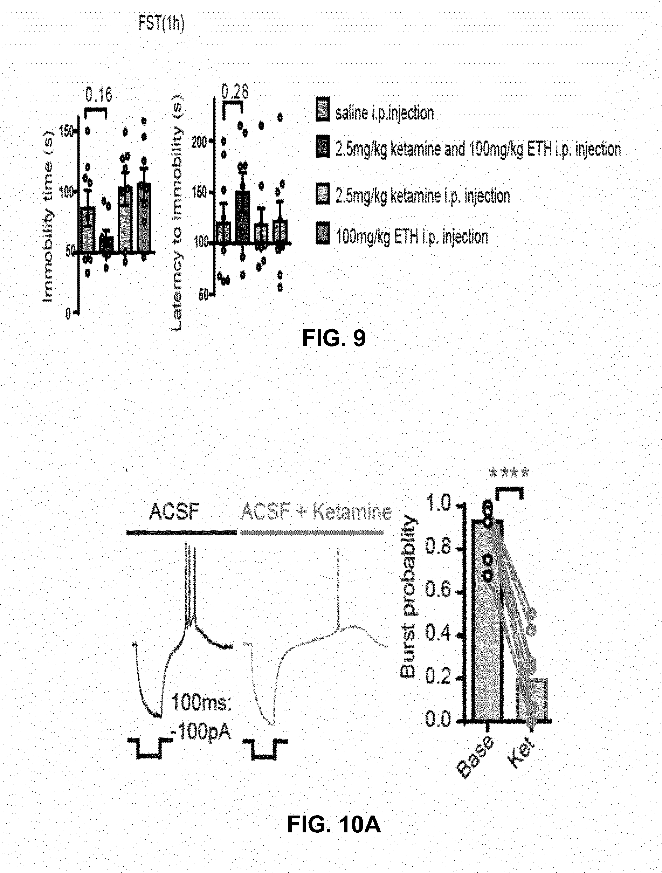

FIG. 9 shows antidepressant-like effect of co-administration of low dose NMDA receptor inhibitor and T-type calcium channel inhibitor in mice. Co-treatment with subeffective doses of both drugs has an antidepressant-like effect.

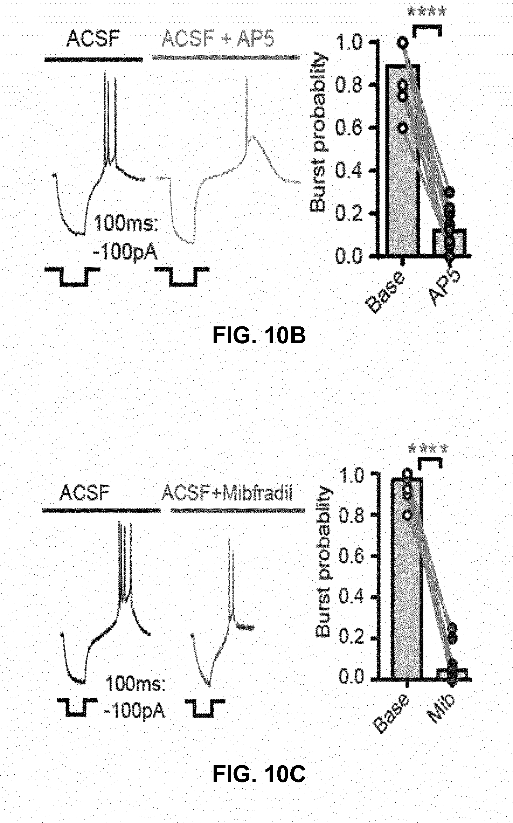

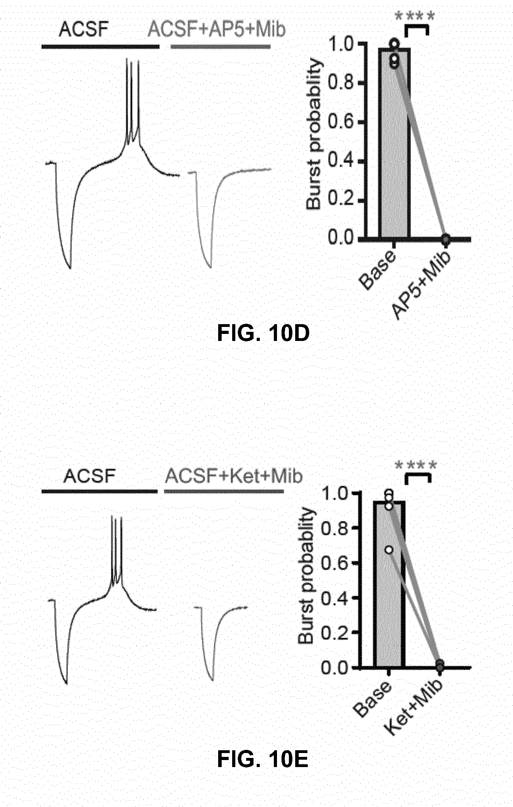

FIG. 10. Effects of co-administration of NMDA receptor inhibitor and T-type calcium channel inhibitor on induction of bursts in LHb. The lateral habenula neurons were injected with -100 pA of hyper-polarizing current for 100 ms, which enable the neurons to be hyperpolarized, and induced the burst firing of lateral habenula neurons. The effects of ketamine (100 µM, A), AP5 (100 µM, B), or Mibefradil (10 µM, C) on the probability of burst firing induced by neurons in the lateral habenula slices are shown. Left is a representative of a burst firing. Statistical analysis is on the right. Effects of Ketamine (100 µM) and Mibefradil (10 µM) in combination (D), or AP5 (100 µM) and Mibefradil (10 µM) in combination (E) burst firing induced by neurons in the lateral habenula slices are shown. Left is a representative of a burst firing. Statistical analysis is on the right. Data are mean ± s.e.m., ****P < 0.0001.

### DETAILED DESCRIPTION

### Example 1 Material and method

Animals. Male cLH rats (4-12 weeks of age) and Sprague Dawley rats (4-12 weeks of age) were used. cLH rats is a selective breeding animal model of depression with congenital learned helpless phenotype (D. Schulz, M. M. Mirrione, F. A. Henn, Neurobiol Learn Mem 93, 291, Feb, 2010). The cLH rats used in this study were introduced from the Malinow Laboratory in Cold Spring Harbor, USA. Feeding and propagation of the cLH rat are described in D. Schulz, et al, Feb, 2010 mentioned above. Rats were group-housed four animals/cage under a 12-h light-dark cycle (light on from 7a.m. to 7p.m.). The cLH rats used in cannula infusion experiment were group-housed one animal/cage. Male adult (8-12 weeks of age) C57BL/6 mice were used in behavior tests; mice were group-housed four animals/cage under a 12-h light-dark cycle (light on from 5a.m. to 5p.m.). Both rats and mice had free access to food and water. All animal studies and experimental procedures were approved by the Animal Care and Use Committee of the animal facility at Zhejiang University.

Viral vectors. AAV9-CaMKII-eNpHR3.0-eYFP (the plasmid was purchased from Addgene, Cat# 26971, packaged by Taitool Bioscience of China); AAV9-Ubi-eGFP (A gift from Guangping Gao Lab, University of Massachusetts, USA); AAV9-hSyn-oChIEF-tdTomato (the plasmid was purchased from Addgene, Cat# 50977, packaged by Taitool Bioscience of China).

### Stereotaxic surgery and histology.

Mice virus injection: Mice were anesthetized by intraperitoneal injection of a mixture of ketamine (100 mg/kg body weight) and xylazine (8mg/kg body weight), and fixed on a stereotactic instrument (Stoelting instruments). Each mouse was injected with 0.1-0.2ul purified and concentrated AAV virus (~ 1013 infection units / ml) on each side of LHb. LHb localization relative to bregma (AP, -1.72 mm; ML, ±0.46 mm; DV, -2.62 mm from the brain surface). Virus injection was performed using a hand-made mircoinjection needle at a slow rate of 100-150nl/min. After the injection was completed, 5 minutes were allowed to pass and leaving it for an additional 5 minutes before the needle was then slowly withdrawn completely.

At least 14 days after injection, mice or rats were used in behavioral or electrophysiological studies. All the injection sites will be checked after the behavioral experiments. Only animals with correct injection site will be counted into the behavioral statistics.

The GFP-labeled virus was checked for GFP protein with antibodies before microscopy. The habenular nucleus area of each brain was cut into 6 groups of consecutive sections (30 µm sections for mice, 6 sections per group; 40 µm sections for rats, 8-9 sections per group). All sections were counterstained with Hoechst prior to mounting on the mount.

Rat bilateral implanted cannula in LHb: Rats were anesthetized by intraperitoneal injection of 4% pentobarbital (60mg / kg body weight) and fixed on stereotaxic apparatus. LHb stereo positioning coordinates for placement of the tube (front and back distance Brema: -3.7mm (AP), left and right side ± 0.7mm (ML), cortical surface down -4.1mm (DV)). Drill in the skull above the place corresponding to LHb, and then three screws were fixed on the skull. Insert the bilateral cannula (purchased from the American Plastics One) into the LHb according to the coordinates, and fix the casing with dental tray cement. After the dental tray cement is completely solidified, insert the casing plug to prevent the plug from falling off. Seven days after surgery, after the rats recover from the surgical trauma, they are used for test the behavioral effects of the drug. After the rats were anesthetized with a gas (isoflurane) anesthesia machine, a cannula matched injection needle was used to slowly inject 1 ul of the drug to be tested (about 100 nl / min) from the cannula, leaving the needle for 10 minutes after the injection, and then removing the injection core. Behavioral tests are carried out based on the effective duration of the drug. After the behavior experiment was completed, CTB-488 or 555 was used to inject from the cannula to check the position of the cannula. Only mice with correct injection site will be counted in behavioral test for statistical analysis.

### In vitro Electrophysiological assay.

Rats (P40-50days) and mice (p8 weeks) were anesthetized with isoflurane, and then perfused with 20 ml ice-cold oxygenated ACSF (118 mM NaCl, 2.5 mM KCl, 26 mM NaHCO₃, 1 mM NaH₂PO₄, 10 mM glucose, 1.3 mM MgCl₂ and 2.5 mM CaCl₂, gassed with 95% O₂ and 5%CO₂). The brain was removed as quickly and put into chilled and oxygenated ACSF. Coronal slices containing habenular (350 µm-thickness) were sectioned in cold ACSF. Slices were allowed to recover for at least 1 hour before recording.

For LHb neuron recordings, a MultiClamp 700B amplifier (Axon Instruments) were used currents were measured under whole-cell patch clamp using pipettes with a typical resistance of 4-6 MΩ filled with internal solution containing (mM) 105 K-Gluconate, 30 KCl, 4 Mg-ATP, 0.3 Na-GTP, 0.3 EGTA, 10 HEPES and 10 Na-phosphocreatine, with pH set to 7.35. The external ACSF solution contained (in mM) 125 NaCl, 2.5 KCl, 25 NaHCO₃, 1.25 NaH₂PO₄, 1 MgCl₂, 2.5 CaCl₂ and 25 Glucose. Cells were visualized with infrared optics on an upright microscope (BX51WI, Olympus). A MultiClamp 700B amplifier and pCLAMP10 software were used for electrophysiology (Axon Instruments). The series resistance and capacitance was compensated automatically after stable Giga seal were formed. The spontaneous neuronal activity was recorded under current-clamp (I=0 pA) for consecutive 60s. RMP was determined during the silent period of the neuronal spontaneous activity.

The record of firing frequency of action potentials in LHb neurons in rats and mice was performed in the I=0 current clamp recording mode for 60 seconds, and the average release frequency was counted in the 60s releases. The different firing modes generated in LHb are defined as: a no-firing cell, which refers to cells that have no action potentials firings during the entire recording process; a single firing cell refers to a discharge frequency between 0.1-10Hz, rarely 10-20Hz; burst firing cells are firings that can be fired in clusters, and the distribution frequency in burst is extremely high, but it shows a gradual decreasing trend, and the frequency of firing is as high as 200 Hz.

Induced NMDA receptor-mediated excitatory post-synaptic currents were recorded at -50mV to -80mV with ACSF at zero magnesium. The induced T-type voltage-sensitive calcium channel current is from cells clamped at -50mV, and then -100mV for 1 second. The stimulus is given at a regular frequency of 0.1 Hz. Calcium current is obtained by linear leakage subtraction.

### In vivo Electrophysiological assay.

Adult male mice were anesthetized by intraperitoneal injection of a mixture of ketamine (100 mg/kg body weight) and xylazine (8mg/kg) and fixed on a stereotactic instrument (Stoelting instruments). A movable electrode array consisting of eight tetrode (composed of four electrode wires) (resistance of 250-500 KS2, California fine wire) was implanted into the LHb (AP: -1.72mm; ML: ± 0.46mm; DV: -2.44mm). The stainless steel wire was wound on two screws fixed to the skull for grounding. The electrodes were fixed to the skull surface with a dental cement. After 5-7 days of recovery, the animal used for recording was started to be trained for adapt for 10 minutes a day for a total of 2-3 days. The 64-channel OmniPlex-D neural signal acquisition system (Plexon Inc., Dallas, TX) was used for recording, and the spontaneous discharge activity of the animal's LHb was recorded in the cage for 30 minutes (sampling frequency 40 kHz, 300-6000 Hz band-pass filtering) and Field potential (LFP, 1kHz sampling rate, 250 Hz low-pass filtering) and gain of 5000. A channel with no discernable neuron firing signal was used as a reference electrode. After each recording, the tetrode steps down at a depth of 70 µm and resumes for 2 days to start the next recording. For CRS mice, the firing activity was recorded 30 min before and 1 hour after ketamine administration. The recorded animals were tested for the electrode site by electrical damage.

Action potential sorting: Import all recorded electrical signals to Offline Sorter V3 (Plexon Inc.), and then manually sort individual neuron discharges using threshold method and principal component analysis (PCA). Emissions with a peak potential interval less than the refractory period (1.4ms) were excluded, and cross-correlation analysis was performed to ensure that unselected neurons did not repeat. Signals that cannot be separated from background noise are eliminated. Data analysis: The software used in this part of the data analysis is Neuroexplorer4 (Plexon Inc.) and MATLAB.

### Behavior tests

### Forced swim test (FST).

The test was performed under normal daylight lamp. Animals were placed in a cylinder (12 cm diameter, 25 cm height). Water depth was 14cm and temperature was 23-24°C. Swimming performance was videotaped from the side for 6 mins. The immobility time during the last 4 min within the 6 min testing period was counted in a double-blind way.

### Sucrose preference test (SPT).

Animals were single housed for a week and habituated with two bottles of water for 2 days, followed by two bottles of 2% sucrose for 2 days. Animals were then exposed to one bottle of 2% sucrose and one bottle of water for 2 h in the testing phase. Bottle positions were switched every 12h. Total consumption of each fluid was measured every 24 hours and for 48 hours in total.

### Chronic restraint stress (CRS) mouse depression model

The animals were randomly divided into two groups. One group was placed in a 50ml centrifuge tube for 2 hours between 11:00 and 14:00 every day for 14 days. Another group of control animals did not receive restraint stress. After each day of restraint, the animals were returned to their cages and housed in the same housing room as the control animals. Forced swimming and sugar water preference tests were performed on day 15 to assess the animal's depressive phenotype.

### Free-moving awake mice optogenetic behavior test

All animal behavior tests in this test are performed when the animal is in the dark period of biorhythm and the virus has expressed at least three weeks. The implanted fiber is connected to the patch cable through a ceramic sleeve (ceramic sleeve purchased from NEWDOON, Hangzhou, China), and the patch cable is connected to a rotating connector (purchased from Doric, Quebec, Canada) through an FC/PC adapter, so that animals can move freely without restriction. Another patch cable is connected to the computer and a 473nm DPSS laser (Aurora-220-473) or a 589nm DPSS laser (Aurora-220-589) via an FC/PC adapter. The lasers were purchased from Newdoon Co. Hangzhou, China.

### Real-time place aversion (RTPA) test

Based on previous described experiments (Matthews et al., 2016; Zhu et al., 2016), a 52 cm x26 cm x23cm open box which is connected in the middle is divided into two boxes of the same size (26 x 26 x 23 cm) for behavioral testing. Mice were placed in the box and allowed to move freely for 20 minutes to assess the basic preference level of the mice to both sides of the box. In the next 20-minute test period, the mice were evenly distributed to the left and right boxes, and one side was used as the stimulation box, and the other side was the non-stimulation box corresponding to each mouse. The mice were placed in a non-stimulation box to begin the experiment. Once the mouse enters the stimulation box, it can activate yellow light stimulation (eNpHR3.0: 589 nm, 1Hz, 16 mW, 100 ms interval) until the mouse returns to the non-stimulated side. A camera is installed directly above the avoidance box to record the activity of each experimental animal. An-maze software (Stoelting, USA) was used to analyze the relevant behavioral indicators of animals. Avoidance score = difference in dwell time between stimulated and non-stimulated side after 20 min - difference between dwell time of stimulated and non-stimulated side in the first 20 min

Open field test: Based on the experimental method of previous research (Matthews et al., 2016), the open field test box size is: 45 x 45 x 45 cm. The four walls and bottom are made of white resin. The animals first explored freely in the open field for a total of 9 minutes, and were given laser stimulation in the middle for 3 minutes (eNpHR3.0: 589nm, 1Hz, 16mW, 100ms interval). The camera set directly above the open field records the movement of each experimental animal, and the related behavioral indicators of the animal are analyzed using Any-maze software.

Forced swimming test: Based on existing forced swimming test methods (Li et al., 2013). The experiments were performed under normal light (about 100 lux). The mice were forced to swim in a cylindrical transparent container with a diameter of 12 cm and a height of 25 cm. The test water depth is 14 cm and the water temperature is 22.5-23 °C. When the mice were placed in water, the laser stimulation started for 6 minutes (eNpHR3.0: 589nm, 1Hz, 16mW, 100ms interval). The camera recorded the swimming of the mice within 6 minutes from the side. After the experiment, double-blind statistics were used to measure the immobility time (the time that the animals were in a floating position or their limbs were completely inactive) of the mice within the last 4 minutes of the 6 minutes of swimming.

### Statistical analyses.

All the data were given as Means±SEM. All behavior testing data were subjected to two-tailed Student's t-tests or Mann-Whitney test.

### Example 2 Local blockade of NMDARs in LHb is sufficient to be rapid antidepressant.

The phenotypic changes of cLH depression rats were observed after ketamine was administered after bilateral cannula implantation. FIG. 1A is a schematic diagram of bilateral cannula implantation in the lateral habenula of a cLH rat, and the white dotted line indicates the position of lateral habenula. The results of the experiment are shown in FIGS. 1B-1G: Local application of different NMDAR inhibitors including ketamine (25 µg each side, FIGS. 1B-1D) and AP5 (40 nmol each side, FIGS. 1E-1G) to LHb can effectively reverse the depression phenotype of cLH rats in a short time ( 0.5 or 1 hour), including significantly reducing immobility time in forced swimming (FIGS. 1C and 1F), and significantly increasing the preference for sugar water in depressed animals (FIGS. 1D and 1G).

It was also observed in the experiment that the antidepressant effect of bilaterally applying the NMDAR inhibitor ketamine in LHb can continue until the 14th day after administration (shown as FIGS. 1H-1I).

The above experiments proved that local inhibition of NMDA receptors in the lateral habenula of rats can achieve rapid and long-lasting antidepressant effects.

This is the first discovery in the field that the administration of NMDA receptor inhibitors, such as ketamine, in local tissues of the brain can produce rapid and long-lasting antidepressant effects.

### Example 3 Distribution characteristics of three types of LHb neurons, the silent, tonic and burst firing types in depressed animals

The whole-cell patch-clamp technique was used to observe the firing pattern of neurons in lateral habenula of depressed animals in isolated brain slices. FIG. 2A shows the recording sites recorded by whole-cell patch-clamp recording, and the recording sites are distributed in different subregions of the lateral habenula. It is found that there are three types of neurons in the lateral habenula, which are silent (shown in FIG. 2B), tonic- (shown in FIG. 2C), and burst- firing (shown in FIG. 2 D).

FIGS. 2E (scatter plot) and 2F (cumulative curve) show the mean and distribution of resting membrane potentials (RMPs). The results showed that compared with silent cells, the resting membrane potential of tonic-firing cells was depolarized and the burst-firing cells appeared to be hyper-polarized.

In addition, experiments have found that in rat and mouse animal models of depression, the proportion of neurons with spontaneous burst firing in the lateral habenula is significantly higher than in control normal animals, and the NMDAR inhibitor ketamine can significantly reduce the ratio of burst firing neurons in depressed animals. This results suggested that the burst firing of lateral habenula neurons in depression animals is enhanced. FIGS. 2I and 2L (pie chart) show that the number of burst firing neurons increased in rat and mouse depression models. FIGS. 2J and 2M (histograms) show the proportion of tonic- and burst-firing cells among all released cells. FIGS. 2K and 2N (histograms) show the distribution of inter-spike intervals in the habenula of the animal with depression.

### Example 4 in vivo test for observation of neuron firing patterns of LHb in depressed animals

In order to further confirm the effect of burst firing of LHb neurons on depression, in vivo multichannel electrophysiological recording method which is a better way to simulate the physiological state of animals, was used in the lateral habenula of awake mice. The multichannel electrophysiology recording electrode records the firing of neurons in the LHb, including mouse burst firing activity and θ-band synchronization. The results are shown in FIG. 3.

FIG. 3A shows recording sites of each tetrode track in LHb of CRS (chronic restrain stress) and control mice. Unlike in in vitro slice conditions where LHb neurons spike with either tonic or bursting mode, spike patterns of LHb neurons recorded in vivo switched between tonic and burst firing modes (FIG. 3B). LHb neurons from CRS mice showed a notable increase in bursting activity (FIG. 3C) but not tonic firing, compared with control naive mice. Injection of ketamine at the antidepressant dosage (10mg/kg, i.p., 1h prior to recording) significantly suppressed the LHb bursting activity (FIG. 3D). The cumulative frequency distributions of ISI (control: 143ms; CRS: 33ms, CRS+ketamine: 121 ms), which were clearly different between CRS and control mice, were significantly shifted toward control level by ketamine (FIG. 3E). The dotted line indicates the point where the peak potential changes by 50%.

Burst firing was known to increase network synchronization. We thus tested whether network synchronization was altered in the LHb network of CRS animals. It was first calculated the spike-triggered averages (STAs) of local field potential (LFP), which revealed oscillatory synchronization between spikes and LFP. In the control mice, the distribution of the power spectra of STAs was relatively flat (FIG. 3F), indicating a lack of synchronization. In the CRS mice, there emerged a dominant frequency of 7Hz in the power spectra of STAs (FIG. 3F), indicating that spikes tended to phase-lock with LFP in the theta-band range (4-10Hz), and this can be blocked by ketamine.

FIG. 3G showed spike-field coherence (SFC) of neurons recorded from control, CRS, and CRS mice after ketamine injection. Left: SFC of each unit; Middle: average SFC; Right: percent SFC in θ band (4-10Hz). The results further proved that LHb network synchronization in CRS mice can be reversed by injection of ketamine.

The above results show that, in the mouse model of chronic restraint stress-induced depression, the frequency and number of inter firings of burst firing in LHb neurons are significantly higher than those of control normal mice. In addition, the increase of the frequency and number of inter firings of burst firing in LHb neurons can be reversed by NMDAR inhibitor ketamine.

### Example 5. In vivo experiment proves that bursts in LHb directly require activation of NMDAR.

Given that NMDAR-mediated calcium influx plays a pivotal role in burst generation in several brain regions, it was tested if NMDARs are directly required for the bursting activity in the LHb. First to confirm that LHb expresses functional NMDARs, NMDAR-dependent excitatory post-synaptic potentials (NMDAR-EPSCs) in sagittal LHb slices was recorded by stimulating the input stria medullaris (SM) fiber in the presence of AMPA receptor (AMPAR) blocker NBQX and GABA receptor (GABAR) blocker picrotoxin and 0 Mg2+, and isolated characteristic NMDAR-currents, which can be abolished by AP5 (FIGS. 4A and 4B).

FIGS. 4C-H show effects of ketamine (C-D), AP5 (E-F) or NBQX (G-H) on spontaneous bursts in LHb. As exemplified by FIGS. 4C and 4D, it was found that ketamine almost completely eliminated spontaneous bursts. As illustrated in FIG. 4C, within seconds after bath application of ketamine, a burst-firing LHb neuron was converted to tonic-firing mode. Similarly, application of a specific NMDAR antagonist AP5 also stopped burst-firing (FIGs. 4E and 4F). Blockade of AMPAR with NBQX (10µM) reduced bursts, but to a much smaller extent than NMDAR blockade (FIGS. 4G and 4H).

To further verify the causal link between NMDAR activity and LHb bursting, we perfused NMDA onto the LHb brain slice. NMDA application induced strong bursting activity in 10 out of 13 originally silent LHb neurons (FIGS. 4I and 4J). Again this bursting activity was blocked by additional bath application of ketamine (FIG. 4J). FIG. 4I shows a representative example of such neurons, exhibiting dramatically enhanced EPSPs and burst firing quickly after perfusion of NMDA.

The results prove that bursts in LHb directly require activation of NMDAR.

### Example 6. Bursts in LHb also depend on hyperpolarization and synergistic activation of T-VSCCs.

Given the correlation between the RMPs and firing mode of LHb neurons, we next tested whether changing RMPs can alter the pattern of spiking activity in LHb. As shown in FIG. 5, by applying a transient ramp-like current injection enabling RMPs to change progressively from around -80 to -40 mV (FIG. 5A). Hyper-polarized current injection causes the cells to give burst firing. The number of inter-sparks in the burst firings was between -56 and -60mV, which was similar to the resting membrane potential of spontaneous burst firing cells. At the same time, hyper- or de-polarized current injection can be given to spontaneous firing cells which can also transform cells between tonic- or burst-firing, and cluster release.

FIG. 5A is a typical diagram of the transformation of LHb neurons from a burst firing cell to a tonic firing cell induced by ramp current injection. As shown in the figure, neurons are prone to burst firing in a relatively hyperpolarized state, and to tonic firing in a relatively depolarized state. FIG. 5B is a statistical graph showing the proportion of neurons that can be induced to burst firing after LHb neurons are injected with hyperpolarization current in rats and mice. FIG. 5C-E show correlations of membrane potential versus inner burst frequency (C), burst duration (D) and inner burst spike number (E) generated by current ramps...

Since the NMDA receptor is a channel that is activated only under depolarized conditions, and the resting membrane potential of neurons that generate burst firing is hyper-polarized, the inventors of the present application then further studied how the NMDA receptor is activated and participate in burst firing under the hyper-polarized condition.

The authors have discovered an ion channel that is activated and depolarizes neurons when the neurons are hyper-polarized: T-type voltage-sensitive calcium ion channels. T-type voltage-sensitive calcium channels are a type of calcium channel that is activated under super-activation. After activation, the channels cause calcium ions to flow in and cause neurons to depolarize. The T-type Voltage Sensitive Calcium Channels (T-VSCCs, including Cav3.1, 3.2, 3.3) are known to have pacemaker activity and are expressed in LHb neurons.

The authors have experimentally demonstrated that the activation of T-type voltage-sensitive calcium ion channels in LHb can trigger burst firing. F in FIG. 5 is a typical example diagram of a spontaneous tonic firing neuron transforming into a burst firing under hyperpolarization. G in FIG. 5 is a typical example diagram of the spontaneous burst firing turned to a tonic firing under depolarization.

It was then tested the effect of T-VSCC on the spontaneous or induced burst firing of LHb. The effect of another ion channel-HCN channel (hyperpolarization-activated cyclic nucleotide-gated channel) on the spontaneous or induced burst firing of LHb was also tested. The experiment was performed by testing the effects of T-VSCC blockers and HCN blockers on the spontaneous or induced burst firing of LHb. FIGS. 5 H and 5I show the effects of the T-VSCC blocker Mibefradil (FIG. 5H) and the HCN channel blocker ZD7288 (FIG. 5I) on the spontaneous burst firing of LHb neurons. A representative graph is given on the left and a statistical graph is given on the right. The results show that T-VSCC blockers can significantly inhibit the frequency of spontaneous or induced burst firing of LHb. The effect of HCN channel blockers on the spontaneous or induced burst firing of LHb is much weaker than that of T-VSCC blockers.

Therefore, the authors discovered for the first time that NMDA receptors and T-VSCCs synergistically cause spontaneous burst firing in the LHb. The physiological process of this burst firing is shown in FIG. 5J: Activated T-VSCC causes the magnesium ions that block NMDA receptors to be removed, and the opening of T-VSCC and NMDA receptor channels drives the neuronal membrane potential to hyperpolarization condition as required for burst firing. When the T-VSCC and NMDA receptor channels are rapidly inactivated, the resting membrane potential of the neuron returns to -55mV, and another cluster discharge cycle is initiated.

Electrophysiological records and model data of these experiments have demonstrated that the T-VSCC and NMDA receptors synergistically mediate burst firing of LHb neurons.

### Example 7. Local blockade of T-VSCCs in LHb is rapidly anti-depressive in animal model

The inventors performed bilateral infusion of a T-VSCC blocker, mibefradil (10 nmol, 1ul each side), in the LHb of cLH rats through dual guide cannulae (FIG. 6A). Mibefradil infusion quickly rescued the depressive-like behaviors, including the immobility in the FST (FIG. 6B) and the anhedonia in the SPT (FIG. 6C) 1hr after infusion.

It proves that local blockade of T-VSCCs in LHb is rapidly anti-depressive.

### Example 8 Increase of burst firing but not the whole firing contribute to the development of depression

The authors have found for the first time that it is the increase of burst firing but not the increase of whole neuron firing contributes to the development of depression.

By activating the inhibitory light-sensitive channel eNpHR3.0, the authors could detect a reversed burst firing on isolated brain slices. At the same time, through in vivo experiments, the authors light-activated the eNpHR3.0 light-sensitive channel expressed in the LHb in animals and found that it can also rapidly mediate the generation of aversion mode and depression phenotype.

FIG. 7A shows a schematic diagram of the construction of the eNpHR virus expression vector (drawing on the top), and a schematic diagram of photoelectricity and recording (drawing on the bottom).

FIGS. 7B and 7C are representative diagrams of yellow light-activated burst rebound in brain slice neurons (FIG. 7B) and in vivo recorded neuronal (FIG. 7C) in mouse whose LHb expresses AAV2/9-eNpHR virus. The percentage of cells that successfully induced a burst firing is shown in the statistical graph on the right side of FIG. 7C. (FIG. 7D) Dot-matrix and post-stimulation time histograms show the response of a representative LHb neuron to 100 ms yellow light stimulation in a body photoelectrode recording. After the yellow light is over, the neurons have a rebound firing frequency. The emission frequency of intra-cluster action potentials generated by 1Hz yellow light irradiation of eNpHR3.0 is equivalent to that recorded in LHb of CRS depressed animals, suggesting that 1Hz yellow light irradiation of eNpHR3.0 can simulate burst firing levels as that in depression condition. At the same time, the results of behavioral studies also found that eNpHR photoactivation-induced rebound burst firing caused animals to exhibit aversive and depressive phenotypes. FIGS. 7F, 7G, and 7H show that the rebound burst firing caused by eNpHR light activation can induce real-time position aversion (RTPA) and depression phenotype. The above results show that increasing the burst firing of LHb is sufficient to produce a depression-like phenotype.

In contrast, using the 5 Hz light to activate the oChIEF optical channel generates a single 5 Hz tonic firing (FIG. 8A), and the total frequency of the firings are equivalent to that cause by the 1 Hz eNpHR 3.0 light activation (one burst firring per second, 5 sparks in each burst, a total firing frequency of 5 Hz in a burst) (FIG. 8B), but no depression phenotype was induced (FIG. 8C). Compared to eGFP mice expressing a control non-light channel, light activation did not change the animal's motility (FIG. 8B).

The above results demonstrate that the increase in burst firing patterns rather than the entire firing requency contributes to the occurrence of depression.

### Example 9. Co-administration of subeffective antidepressant doses of NMDAR antagonist and T-VSCC antagonist has an antidepressant-like effect.

Low dose ketamine (one of the NMDAR antagonists, 2.5 mg/kg) or ethosuximide (ETH, one of the T-VSCC antagonists, 100 mg/kg) is ineffective in the forced swim test in C56BL/6 mice 1 hour after drug administration (i.p.) (FIG. 9). In co-administration of 2.5 mg/kg of ketamine and 100 mg/kg ethosuximide, as sub effective antidepressant doses of either drug, the immobility time was reduced and latency to immobility was increased compared to control group (FIG. 9). Hence, Co-administration of subeffective antidepressant doses of ketamine and ethosuximide could an antidepressant-like effect.

These above in vivo experiments suggest that administering an NMDA receptor inhibitor at a dosage lower than its working dosage in combination with administering a T-VSCC receptor inhibitor at a dosage lower than working dosage, could be effective in generating a significant anti-depression effect.

### Example 10. Pharmacological manipulations of hyperpolarization-triggered rebound bursts in LHb.

To further test the effect of NMDAR inhibitors combined with T-VSCC inhibitors on the induction of burst firing, the effects on inducing burst firing by NMDAR inhibitors and T-VSCC inhibitors alone or in combination were tested.

Based on the observation that a hyperpolarization ramp current could induce burst firing in the LHb, a protocol was devised employing a transient (100 ms) hyperpolarization current injection, which induced rebound bursts in the LHb brain slices with 100% success rate (FIGS. 10A-10E). As seen, -100 pA hyperpolarization current was injected into the lateral habenular neurons for 100 ms, which made the neurons hyperpolarized, and the hyperpolarization state induced burst firings of the lateral habenular neurons.

When the lateral habenular slices were perfused with ketamine (100 µM, FIG. 10A), AP5 (100 µM, FIG. 10B) or mibefradil (10 µM, FIG. 10C), the probability of neuron hyperpolarization-induced burst was partially reduced to 0.19, 0.12 and 0.05, respectively. When AP5 (100 µM) was administered in combination with mibefradil (10 µM, FIG. 10D) or ketamine (100 µM) was administered in combination with mibefradil (10 µM, FIG. 10E), the probability of neuron hyperpolarization- induced burst in both tests were further reduced to almost zero, which means a complete blocking of the hyperpolarization-induced burst.

Thus in other words, the hyperpolarization-induced rebound bursts can be partially inhibited by ketamine (100 µM, FIG. 10A), AP5 (100 µM, FIG. 10B), or mibefradil (10 µM, FIG. 10C), but can be almost fully blocked by mibefradil (10 µM) in combination with ketamine (100 µM, FIG. 10E) or AP5 (100 µM, FIGS. 10D), indicating that the combination of NMDAR antagonists and T-VSCC antagonists has a strong synergy in modulating the hyperpolarization-induced rebound bursts.

Collectively, these above in vitro experiments demonstrate that a combination of a low dose of an NMDA receptor inhibitor that is lower than the effective dose when administered alone and a low dose of a T-VSCC receptor inhibitor that is also lower than the effective dose when administered alone can produce a much more pronounced and significant antidepressant effect. It is also noteworthy that since the lower than effective doses of the NMDA receptor inhibitor and the T-VSCC receptor inhibitor are administered, there is potentially another benefit for a reduced side effect for both drugs.

Unless otherwise indicated herein, the temperature unit "degrees" refers to Celsius degrees, namely °C.

## Claims

1. A pharmaceutical composition for use in a method of treating depression in a subject, wherein the depression is **characterized by** having an increased burst firing in neurons of a lateral habenula of the subject, wherein the pharmaceutical composition is administered locally into the lateral habenula of the subject, the pharmaceutical composition comprising a reagent that is capable of inhibiting the burst firing, but is incapable of inhibiting tonic pulse, in the neurons of the lateral habenula, wherein the reagent comprises one selected from:
ketamine;
AP5;
Mibefradil;
a combination of ketamine and Mibefradil; or
a combination of AP5 and Mibefradil.

2. The pharmaceutical composition for use according to Claim 1, wherein the reagent of the pharmaceutical composition comprises ketamine.

3. The pharmaceutical composition for use according to Claim 1, wherein the reagent of the pharmaceutical composition comprises AP5.

4. The pharmaceutical composition for use according to Claim 1, wherein the reagent of the pharmaceutical composition comprises Mibefradil.

5. The pharmaceutical composition for use according to Claim 1, wherein the reagent of the pharmaceutical composition comprises a combination of ketamine and Mibefradil.

6. The pharmaceutical composition for use according to Claim 1, wherein the reagent of the pharmaceutical composition comprises a combination of AP5 and Mibefradil.

7. A pharmaceutical composition for use in a method of treating depression in a subject, wherein the depression is **characterized by** having an increased burst firing in neurons of a lateral habenula of the subject, wherein the pharmaceutical composition is administered locally into the lateral habenula of the subject, the pharmaceutical composition comprising a reagent that is capable of inhibiting the burst firing, but is incapable of inhibiting tonic pulse, in the neurons of the lateral habenula, wherein the reagent comprises a combination of ketamine and ethosuximide, wherein ketamine has a dose of 2.5 mg/kg and ethosuximide has a dose of 100 mg/kg.

## Patentansprüche

1. Pharmazeutische Zusammensetzung zur Verwendung in einem Verfahren zur Behandlung von Depression in einem Subjekt, wobei die Depression **dadurch gekennzeichnet ist, dass** sie eine erhöhte Burst-Feuerung in Neuronen einer lateralen Habenula des Subjekts aufweist, wobei die pharmazeutische Zusammensetzung lokal in die laterale Habenula des Subjekts verabreicht wird, wobei die pharmazeutische Zusammensetzung ein Reagenz umfasst, das in der Lage ist, die Burst-Feuerung zu hemmen, aber nicht in der Lage ist, den tonischen Puls in den Neuronen der lateralen Habenula zu hemmen, wobei das Reagenz eines umfasst, das ausgewählt ist aus:
Ketamin;
AP5;
Mibefradil;
einer Kombination von Ketamin und Mibefradil; oder
einer Kombination von AP5 und Mibefradil.

2. Pharmazeutische Zusammensetzung zur Verwendung nach Anspruch 1, wobei das Reagenz der pharmazeutischen Zusammensetzung Ketamin umfasst.

3. Pharmazeutische Zusammensetzung zur Verwendung nach Anspruch 1, wobei das Reagenz der pharmazeutischen Zusammensetzung AP5 umfasst.

4. Pharmazeutische Zusammensetzung zur Verwendung nach Anspruch 1, wobei das Reagenz der pharmazeutischen Zusammensetzung Mibefradil umfasst.

5. Pharmazeutische Zusammensetzung zur Verwendung nach Anspruch 1, wobei das Reagenz der pharmazeutischen Zusammensetzung eine Kombination von Ketamin und Mibefradil umfasst.

6. Pharmazeutische Zusammensetzung zur Verwendung nach Anspruch 1, wobei das Reagenz der pharmazeutischen Zusammensetzung eine Kombination von AP5 und Mibefradil umfasst.

7. Pharmazeutische Zusammensetzung zur Verwendung in einem Verfahren zur Behandlung von Depression in einem Subjekt, wobei die Depression **dadurch gekennzeichnet ist, dass** sie eine erhöhte Burst-Feuerung in Neuronen einer lateralen Habenula des Subjekts aufweist, wobei die pharmazeutische Zusammensetzung lokal in die laterale Habenula des Subjekts verabreicht wird, wobei die pharmazeutische Zusammensetzung ein Reagenz umfasst, das in der Lage ist, die Burst-Feuerung zu hemmen, aber nicht in der Lage ist, den tonischen Puls in den Neuronen der lateralen Habenula zu hemmen, wobei das Reagenz eine Kombination aus Ketamin und Ethosuximid umfasst, wobei das Ketamin eine Dosis von 2,5 mg/kg aufweist und das Ethosuximid eine Dosis von 100 mg/kg aufweist.

## Revendications

1. Composition pharmaceutique pour utilisation dans un procédé de traitement de dépression chez un sujet, la dépression étant
**caractérisée en ce qu'**elle a une décharge en rafale accrue dans des neurones d'une habénula latérale du sujet, la composition pharmaceutique étant administrée localement dans l'habénula latérale du sujet, la composition pharmaceutique comprenant un réactif qui est capable d'inhiber la décharge en rafale, mais est incapable d'inhiber une impulsion tonique, dans les neurones de l'habénula latérale, le réactif comprenant l'un choisi parmi :
kétamine ;
AP5;
Mibéfradil ;
une combinaison de kétamine et de Mibéfradil ; ou
une combinaison d'AP5 et de Mibéfradil.

2. Composition pharmaceutique pour utilisation selon la revendication 1, dans laquelle le réactif de la composition pharmaceutique comprend de la kétamine.

3. Composition pharmaceutique pour utilisation selon la revendication 1, dans laquelle le réactif de la composition pharmaceutique comprend de l'AP5.

4. Composition pharmaceutique pour utilisation selon la revendication 1, dans laquelle le réactif de la composition pharmaceutique comprend du Mibéfradil.

5. Composition pharmaceutique pour utilisation selon la revendication 1, dans laquelle le réactif de la composition pharmaceutique comprend une combinaison de kétamine et de Mibéfradil.

6. Composition pharmaceutique pour utilisation selon la revendication 1, dans laquelle le réactif de la composition pharmaceutique comprend une combinaison d'AP5 et de Mibéfradil.

7. Composition pharmaceutique pour utilisation dans un procédé de traitement de dépression chez un sujet, la dépression étant
**caractérisée en ce qu'**elle a une décharge en rafale accrue dans des neurones d'une habénula latérale du sujet, la composition pharmaceutique étant administrée localement dans l'habénula latérale du sujet, la composition pharmaceutique comprenant un réactif qui est capable d'inhiber la décharge en rafale, mais est incapable d'inhiber une impulsion tonique, dans les neurones de l'habénula latérale, le réactif comprenant une combinaison de kétamine et d'éthosuximide, la kétamine ayant une posologie de 2,5 mg/kg et l'éthosuximide ayant une posologie de 100 mg/kg.
